(19) Europäisches Patentamt
European Patent Office
Office européen des brevets

(11) **EP 2 784 504 A1**

(12) **DEMANDE DE BREVET EUROPEEN**

(43) Date de publication:
**01.10.2014 Bulletin 2014/40**

(51) Int Cl.:
***G01N 33/00*** *(2006.01)*

(21) Numéro de dépôt: **14305326.2**

(22) Date de dépôt: **06.03.2014**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **25.03.2013 FR 1352656**

(71) Demandeur: **IFP Energies nouvelles**
**92852 Rueil-Malmaison Cedex (FR)**

(72) Inventeur: **Rouchon, Virgile**
**92210 Saint-Cloud (FR)**

(54) **Procédé et système d'analyse d'un fluide gazeux comprenant au moins un gaz rare au moyen d'un substrat de getterisation**

(57)    L'invention concerne un procédé et un système d'analyse des gaz rares présents dans un fluide gazeux (1). Selon l'invention, dans un premier temps, on extrait par piégeage au moyen d'un substrat de getterisation (5) les gaz rares du fluide gazeux, puis on réalise une surconcentration des gaz rares avant injection (8) dans les instruments de mesure (9). Grâce à l'invention, on peut augmenter la pression partielle des gaz rares dans les gaz à analyser avant leur injection dans les instruments d'analyse.

Figure 1

**Description**

La présente invention concerne le domaine de l'analyse des gaz, notamment des gaz prélevés dans des réservoirs géologiques.

Afin de connaître au mieux les réservoirs géologiques, il est intéressant de déterminer la composition des gaz qui y sont contenus. Ces gaz comprennent notamment des gaz rares non radioactifs (également appelés gaz nobles ou gaz inertes) : hélium He, néon Ne, argon Ar, krypton Kr, xénon Xe. Il s'agit d'éléments chimiques très peu réactifs, appartenant au groupe 18 du tableau périodique des éléments, sous forme de gaz dans des conditions normales de température et de pression. Ils sont monoatomiques incolores et inodores. Ces gaz sont dits inertes car ils sont quasiment dépourvus de réactivité chimique.

L'étude des gaz rares dans le domaine des gaz naturels permet de contribuer à la compréhension de l'origine du gaz naturel, et des processus naturels ayant entrainé son évolution au sein des réservoirs géologiques. Cette évolution se caractérise généralement par la superposition de différents processus chimiques, biologiques et physiques, qui vont engendrer des modifications de la composition élémentaire et isotopique du gaz, dont notamment celle des gaz rares. On peut citer notamment le très grand intérêt de l'isotopie de l'He pour tracer l'origine du $CO_2$ dans les bassins sédimentaires, crustale ou mantélique, et de l'intérêt des fractionnements élémentaires de certains isotopes du Ne, de l'Ar et du Kr pour identifier les différents équilibres de phases (eau, huile, gaz) ayant marqué l'histoire d'un fluide (exemple : brevet US 4833915). Dans le cadre du stockage de gaz, les gaz rares sont d'efficaces traceurs de fuite et peuvent être à la base de calculs de bilans de masse de l'état chimique du gaz stocké (exemple : brevets WO2010-049608 ; FR 2 938 068).

Dans la perspective de mise en application à l'échelle industrielle de l'analyse des gaz rares dans le domaine pétrolier et gazier, un besoin de développement d'un système d'analyse compacte, robuste et rapide est nécessaire. Connaissant les contraintes analytiques sur les gaz rares (abondances, isotopie, volumes d'échantillon, précision analytique nécessaire), des développements ont porté sur une analyse par chromatographie en phase gazeuse couplée à une spectrométrie de masse. Ces instruments sont largement disponibles dans le commerce et leur fiabilité n'est plus à démontrer. En revanche, la faible abondance des gaz rares dans la nature, spécifiquement dans le domaine des hydrocarbures, requiert un pré-traitement des gaz pour préparer les gaz rares aux conditions d'analyse (volume, pression...), ce qui nécessite un appareillage volumineux et souvent réalisé sur mesure par des laboratoires compétents. Aujourd'hui ces systèmes sont des lignes de préparation sous ultravide, couplés à des spectromètres de masse à secteurs magnétiques ou quadripolaire.

L'invention concerne un procédé et un système d'analyse des gaz rares présents dans un fluide gazeux. Selon l'invention, dans un premier temps, on extrait par piégeage au moyen d'un substrat de getterisation les gaz rares du fluide gazeux, puis on réalise une surconcentration des gaz rares avant injection dans les instruments de mesure. Grâce à l'invention, on peut augmenter la pression partielle des gaz rares dans les gaz à analyser avant leur injection dans les instruments d'analyse. Ainsi, les avantages se trouvent principalement dans le fait que l'invention donne la possibilité d'utiliser des instruments d'analyse plus commun que ceux utilisés aujourd'hui pour l'analyse des gaz rares, et donc par une diminution des coûts de maintenance, la simplicité du procédé et du système.

**Le procédé et le système selon l'invention**

L'invention concerne un procédé d'analyse d'un fluide gazeux comprenant au moins un gaz rare. Pour ce procédé, on réalise les étapes suivantes :

a) on crée le vide dans une chambre d'accumulation comprenant un substrat de getterisation, ledit substrat de getterisation étant apte à piéger les composants dudit fluide gazeux à l'exception dudit gaz rare ;
b) on contrôle le débit dudit fluide entrant dans ladite chambre d'accumulation au moyen d'un restricteur de débit ;
c) on extrait et on accumule ledit gaz rare dudit fluide gazeux par passage dudit fluide gazeux dans ledit substrat de getterisation de ladite chambre d'accumulation;
d) on comprime ledit gaz rare extrait de sorte que la pression et le volume dudit gaz rare comprimé soient adaptés à la pression et au volume de ladite analyse ; et
e) on analyse ledit gaz rare comprimé.

Selon l'invention, on analyse ledit gaz rare par chromatographie en phase gazeuse couplée à une spectrométrie de masse.

Avantageusement, ledit substrat de getterisation est un alliage métallique sous forme de mousse, de poudre, de dépôt, de tiges, de rubans.

De préférence, ledit substrat de getterisation est une mousse ou une poudre de titane, de zirconium, d'aluminium ou d'alliage de l'un de ces métaux.

Selon un mode de réalisation de l'invention, on chauffe ledit substrat de getterisation pour l'étape d'extraction dudit gaz rare.

En outre, préalablement à ladite étape d'extraction dudit gaz rare, on peut réaliser le vide dans une chambre intégrant ledit substrat de getterisation.

De plus, on peut contrôler le débit du passage dudit fluide gazeux dans ledit substrat de getterisation.

L'invention concerne également un système d'analyse d'au moins fluide gazeux comprenant au moins un gaz rare. Le système comprend :

- un restricteur de débit pour contrôler le débit dudit fluide gazeux,
- des moyens d'extraction et d'accumulation dudit gaz rare comprenant une chambre d'accumulation intégrant un substrat de getterisation, ledit substrat de getterisation étant apte à piéger tous les composants dudit fluide gazeux à l'exception dudit gaz rare,
- des moyens de mise sous vide de ladite chambre d'accumulation,
- des moyens de compression dudit gaz rare extrait, les moyens de compression étant aptes à comprimer et à injecter ledit gaz rare pour ladite analyse, et
- des moyens d'analyse dudit gaz rare comprimé.

De manière avantageuse, ledit fluide gazeux est un échantillon de gaz prélevé dans un réservoir géologique.

Avantageusement, lesdits moyens d'analyse dudit gaz rare comprennent un chromatographe couplé à un spectromètre de masse.

Selon un mode de réalisation de l'invention, ledit substrat de getterisation est un alliage métallique sous forme de mousse, de poudre, de dépôt, de tiges ou de rubans.

De préférence, ledit substrat de getterisation est une mousse ou une poudre de titane, de zirconium, d'aluminium ou d'alliage de l'un de ces métaux.

De plus, ledit système d'analyse peut comprendre en outre des moyens de chauffage de ladite chambre intégrant ledit substrat de getterisation.

Selon l'invention, ledit système d'analyse comprend en outre des moyens de mise sous vide de ladite chambre apte à faire le vide dans ladite chambre intégrant ledit substrat de getterisation, lesdits moyens de mise sous vide comprenant au moins une pompe.

Avantageusement, lesdits moyens de mise sous vide comprennent en outre un deuxième substrat de getterisation apte à piéger les molécules gazeuses réactives présentes dans ladite chambre sous vide poussé.

Selon une variante de réalisation de l'invention, lesdits moyens de compression dudit gaz rare comprennent une pompe seringue de surconcentration comportant un piston dont le déplacement peut être commandé pour adapter le volume et la pression dudit gaz rare pour ladite analyse.

De plus, ledit système d'analyse peut comprendre une pompe seringue de transfert apte à capter ledit fluide gazeux et à le transférer auxdits moyens d'extraction dudit gaz rare, ladite pompe seringue de transfert comprenant un piston dont le déplacement peut être commandé pour adapter le débit d'injection dudit fluide dans lesdits moyens d'extraction dudit gaz rare.

Par ailleurs, ledit système d'analyse peut comporter un restricteur de débit en amont des moyens d'extraction dudit gaz rare.

**Présentation succincte des figures**

D'autres caractéristiques et avantages du procédé selon l'invention, apparaîtront à la lecture de la description ci-après d'exemples non limitatifs de réalisations, en se référant à la figure annexée et décrite ci-après.

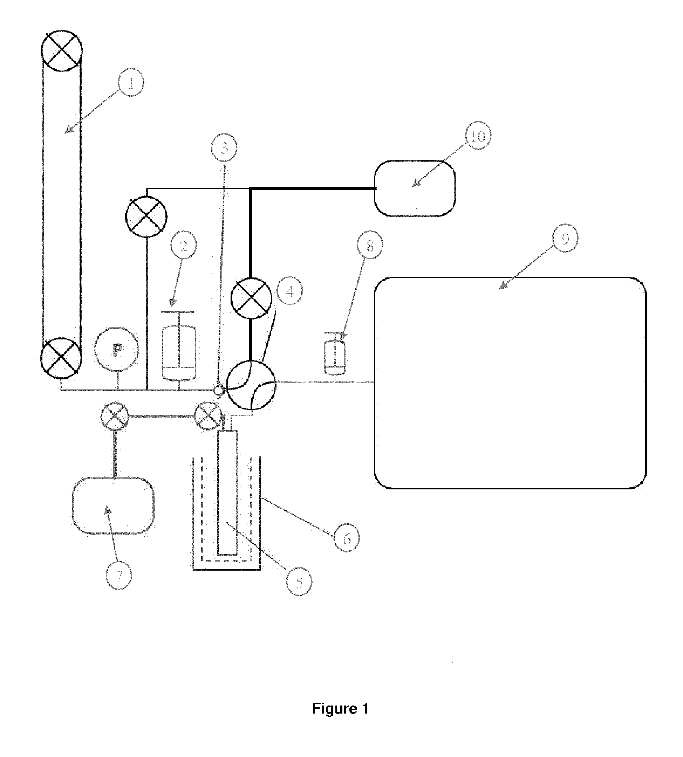

La figure 1 illustre un mode de réalisation du système d'analyse selon l'invention.

**Description détaillée de l'invention**

L'invention concerne un procédé d'analyse d'un fluide gazeux, le fluide gazeux comprenant au moins un gaz rare. Le procédé selon l'invention permet de déterminer, de caractériser, et de quantifier les gaz rares présents dans le fluide gazeux. De préférence, le fluide gazeux correspond à un mélange gazeux prélevé dans un réservoir géologique contenant des hydrocarbures. Avantageusement, ce fluide de gaz est un échantillon du mélange gazeux, l'échantillon peut être contenu dans un récipient de forme sensiblement cylindrique, dont les extrémités sont équipées d'au moins une vanne pour libérer et/ou enfermer un gaz dans le récipient.

Le procédé d'analyse d'un fluide gazeux selon l'invention comporte les étapes suivantes :

1. Extraction des gaz rares
2. Compression des gaz rares
3. Analyse des gaz rares

1. Extraction des gaz rares

Cette étape permet de séparer les gaz rares des autres constituants du fluide gazeux (c'est-à-dire les espèces gazeuses réactives, par opposition aux gaz rares qui sont inertes). Pour cela on injecte le fluide gazeux à analyser dans des moyens de piégeage des autres constituants du fluide gazeux, qui permettent d'extraire uniquement les gaz rares, en piégeant les autres constituants du fluide gazeux. Dans le cas d'un fluide gazeux du domaine gazier ou pétrolier, les autres constituants sont essentiellement des hydrocarbures, du $CO_2$ et de l'azote.

Selon l'invention, l'extraction des gaz rares est réalisée au moyen d'un substrat de getterisation. Un getter est une substance utilisée pour extraire par combinaison ou par adsorption des quantités, généralement faibles, d'autres substances d'un fluide. Par exemple, un métal tel que le magnésium peut être utilisé pour extraire les dernières traces d'air lors de la réalisation d'un vide poussé. Différents getters sont également employés pour extraire les impuretés des semi-conducteurs. On parle de substrat car il s'agit d'une substance qui réagit avec un réactif (gaz rare) pour former un produit (getter chargé par les gaz piégés).

Le substrat de getterisation selon l'invention est du type getter non-évaporable pour la purification de gaz sous pression, qui par la réactivité d'un alliage métallique permet de piéger d'une manière irréversible et à volume constant les molécules gazeuses réactives en trace présentes dans un flux de gaz inerte sous pression. Après passage dans le substrat de getterisation, il est possible qu'il reste quelques traces d'autres gaz que les gaz rares, ces traces sont en quantité réduite et ne gênent pas l'analyse.

Avantageusement, le substrat de getterisation selon l'invention est un alliage métallique sous forme de mousse, de poudre, de dépôts, de tiges, de rubans. Cette liste n'est pas exhaustive. De préférence, le substrat de getterisation est une mousse ou une poudre à base de titane, de zirconium, d'aluminium ou à base d'un alliage de ces différents métaux. En effet, ce type de substrat possède une capacité de piégeage élevée.

L'efficacité de piégeage du principe de getterisation peut se chiffrer en mol/cm$^3$ normalisé au diazote. Ce taux de piégeage définit la quantité de gaz piégeable par unité de volume de gaz libre dans une chambre intégrant le substrat de getterisation. La notion volumique ne concerne donc pas le volume occupé par le substrat de getterisation mais par sa "porosité connectée". Cette efficacité de piégeage est donc une mesure du rapport nombre de mol de gaz piégé (Np)/volume de gaz libre (Nl), dans le volume total du getter (NFoG). Afin d'appréhender ce taux de piégeage, on doit considérer la forme (poudre, mousse, dépôt, tiges, rubans...) et la géométrie (sphérique, diamètre...) de l'alliage métallique qui constitue le substrat du getter, ainsi que le potentiel de piégeage brut de cet alliage en mol de gaz piégé par gramme d'alliage de getter.

On trouve peu de valeurs de potentiel de piégeage pour des molécules autres que le dihydrogène dans la littérature. Par exemple, la littérature fait état, pour un alliage Zr-V-Fe, d'un potentiel de piégeage mesuré en flux continu d'éthylène sous pression de 160 $\mu$mol de carbone par gramme d'alliage.

Des tests effectués par le demandeur en laboratoire montrent qu'un substrat de mousse de titane peut avoir un potentiel de piégeage supérieur à celui donné dans la littérature, de l'ordre de 1000 $\mu$mol/g.

La valeur de 1000 $\mu$mol de carbone peut être convertie en cm$^3$ de gaz à pression standard, ce qui donne 20 cm$^3$. Ainsi, un gramme de getter réalisé sous forme de mousse de titane est en mesure de piéger jusqu'à 20 cm$^3$ de gaz réactif monoatomique à pression atmosphérique. En partant d'une poudre à géométrie de grain sphérique de rayon unique, on peut calculer une porosité modèle pour un volume de réacteur rempli par cette poudre d'alliage. Cette porosité, selon l'assemblage des particules, sera comprise dans la gamme 0,50-0,25. On peut donc estimer une valeur haute de taux de piégeage de l'ordre de 1,41.10$^2$ mol.cm$^{-3}$, ou ramené en rapport volumique égale à 346. On peut donc espérer enrichir en gaz rare un gaz initial par un facteur d'environ 350. Ce facteur d'enrichissement ne dépend pas du volume du piège, et ne dépend que des propriétés chimiques et géométriques du substrat de getterisation. Cette valeur correspond au nombre de moles d'atomes piégés, il faut donc la pondérer à la complexité moléculaire du gaz purifié pour déterminer un facteur d'enrichissement effectif. Pour l'air, donc les molécules majoritaires sont di-atomiques ($N_2$, $O_2$), il faut diviser le facteur par deux, pour du $CO_2$ par trois...

L'invention permet de concentrer les gaz rares présents en faibles quantités dans une source de gaz grâce à l'accumulation des gaz rares dans une chambre constituée d'un getter non évaporable. Selon un mode de réalisation de l'invention, la chambre intégrant le substrat de getterisation peut être mise initialement sous vide. Selon l'invention, on contrôle le débit du fluide gazeux pour son passage dans le susbtrat de getterisation, pour cela le fluide gazeux passe préalablement au travers d'un restricteur de débit. Les espèces réactives constitutives de la majorité de la molarité du gaz à traiter vont réagir avec l'élément getter après leur passage d'un restricteur de débit. La purification instantanée des espèces réactives du gaz permettent de maintenir un gradient de pression au travers du restricteur de débit, et l'accumulation des gaz rares dans la chambre à accumulation jusqu'à saturation du getter, ou jusqu'à l'atteinte d'un équilibre de pression de part et d'autre du restricteur de débit. Ainsi, le gaz accumulé dans la chambre intégrant le substrat de getterisation possède une pression partielle de gaz rares supérieure à celle du gaz initiale, selon la loi suivante : $P_i^{GR} \cdot V_i^{GR} = P_f^{GR} \cdot V_f^{GR}$ avec $P_i^{GR}$ pression initiale des gaz rares, $V_i^{GR}$ le volume initial des gaz rares, $P_f^{GR}$ la pression final (c'est-à-dire dans la chambre intégrant le substrat de getterisation) des gaz rares, $V_f^{GR}$ le volume final (c'est-à-dire dans la chambre intégrant le substrat de getterisation) des gaz rares.

En partant du principe que la totalité du volume $V_i$ (volume initial du fluide gazeux, par exemple volume de l'échantillon de gaz) est transféré dans le volume $V_f$ (volume gazeux dans la chambre intégrant le substrat de getterisation) et en adoptant le principe de conservation de la matière, on peut écrire une relation du type :

$$P_f^{GR} = P_i^{GR} \cdot \frac{V_i^{GR}}{V_f^{GR}}.$$

Le facteur d'enrichissement en gaz rares $\dfrac{P_f^{GR}}{P_i^{GR}}$ est donc proportionnel au rapport des volumes $\dfrac{V_i^{GR}}{V_f^{GR}}$, qui peut être choisi par l'utilisateur, moyennant la prise en compte de contraintes mécaniques et de performance du getter. Ainsi, la pression partielle totale en gaz rares d'un échantillon de gaz peut-être augmentée d'une manière très significative, au moyen du substrat de getterisation.

Cette étape permet donc d'extraire les gaz rares du fluide gazeux en augmentant la pression partielle des gaz rares.

Une fois le getter dimensionné, on peut s'intéresser aux aspects temporels qui vont régir le principe général du getter. Un facteur à prendre en compte est clairement la cinétique de piégeage par getterisation. En effet, ce principe repose sur une réactivité de l'alliage en surface avec la phase gazeuse. Cette réaction de surface atteint la saturation lorsque la couche externe de l'alliage a entièrement réagi par des réactions de type :

$$2M + N2 \rightarrow 2MN$$

où M correspond à une mole d'alliage, et N une mole d'azote. Lorsque la couche de surface de l'alliage est saturée, la réaction peut se poursuivre grâce à la diffusion de l'alliage oxydé dans la matrice métallique, et donc la réapparition en surface de l'alliage de métal natif (donc réactif). Le processus de diffusion est plus lent que la cinétique de la réaction en surface. C'est donc cette diffusion qui peut constituer un facteur limitant de la cinétique globale (donc ramenée à la masse d'alliage) de piégeage. Dans cette problématique cinétique, on comprend bien que les aspects de forme et de surface spécifique des particules d'alliage jouent un rôle important. Une surface spécifique du getter ($m^2$/g) maximisée permet de limiter au maximum le facteur limitant de la diffusion. En revanche, une forte surface spécifique augmente très largement la porosité du getter, ce qui pénalise le facteur de préconcentration tel que défini précédemment. Par ailleurs, la température de l'alliage joue un rôle important sur les vitesses de diffusion dans l'alliage. On peut donc optimiser le choix de la forme du substrat de getterisation (l'alliage) ainsi que sa température de fonctionnement afin d'optimiser l'étape d'extraction selon un trio de critères de performance : 1) taux d'enrichissement, 2) temps de mise en oeuvre, 3) encombrement, tout en respectant un cahier des charges quantitatif (quantité d'échantillon initial disponible, quantité d'échantillon nécessaire pour l'analyse, non-contamination, etc...).

Donc, afin de permettre cette optimisation, le procédé selon l'invention peut comprendre une étape de chauffage de la chambre comprenant le substrat de getterisation, lors de l'étape de piégeage.

Un deuxième aspect cinétique concerne la vitesse de "pompage" du getter. En effet, le principe de préconcentration repose sur un différentiel de pression entre le getter et l'arrivée du fluide gazeux. Selon une variante de réalisation, on peut limiter le débit de circulation du fluide gazeux pour l'étape d'extraction des gaz rares. Cette limitation peut être mise en oeuvre par un restricteur de débit, qui assure un différentiel de pression entre l'arrivée du fluide gazeux et le getter. Le dimensionnement du restricteur de débit permet d'engendrer un débit vers le getter d'un ordre de grandeur similaire au taux de piégeage du getter afin d'optimiser la préconcentration et la vitesse de piégeage.

## 2. Compression des gaz rares

Le gaz extrait du fluide gazeux est ensuite comprimé, de sorte que la pression et le volume des gaz rares soient adaptés à la pression et au volume de l'analyse, c'est-à-dire aux appareils de mesure, qui est mise en oeuvre pour déterminer, quantifier et caractériser les gaz rares présents dans le fluide gazeux.

Selon un mode de réalisation de l'invention, on cherche à atteindre un objectif cible de facteur d'enrichissement compris entre 100 et 1000, entre la pression partielle des gaz rares dans le fluide gazeux et la pression partielle des gaz rares après l'étape de compression. Pour atteindre cet objectif, il convient de bien caractériser la capacité de piégeage du substrat de getterisation, et d'adapter à ces résultats une compression des gaz rares, notamment par un dispositif de surconcentration. Ce dispositif de surconcentration peut être constitué d'une pompe seringue par exemple, via un principe d'augmentation de pression du gaz purifié et préconcentré en sortie du getter. Le dimensionnement du dispositif de surconcentration doit intégrer la notion de quantité d'échantillon de gaz nécessaire pour effectuer l'analyse in fine.

Par exemple, pour une application de mesure élémentaire et isotopique des gaz rares, il est possible d'envisager des mesures en chromatographie en phase gazeuse couplée à une spectrométrie de masse (GC-MS) et/ou en spectrométrie de masse statique (MS statique (quadripolaire par exemple)). Une analyse en chromatographie en phase gazeuse couplée à une spectrométrie de masse avec un ratio isotropique (GC-IRMS) pour l'isotopie du carbone par exemple consomme au maximum pour chaque injection de l'ordre de 10 $\mu$L à pression atmosphérique d'un gaz 100 % $CO_2$. Considérant la dilution de gaz rares peu abondants (Ne, Kr, Xe) dans des gaz rares majoritaires (He, Ar) d'un facteur pouvant aller jusqu'à $10^6$ dans des cas extrêmes, il nous faut augmenter la quantité d'échantillon à injecter tout en envisageant des méthodes de détection MS adaptées (détecteurs de gammes différentes pour les différents gaz rares et isotopes). Pour des raisons chromatographiques, l'injection de plus de 500 $\mu$L semble problématique. On peut donc partir d'une injection unitaire d'un volume de 100 $\mu$L. Pour des raisons statistiques, trois injections sont souhaitables pour chaque échantillon, plus un aliquote de gaz pour l'analyse isotopique de l'He (optionnel selon les solutions analytiques finales). Une quantité de 400 $\mu$L semble donc être un minimum. Par conséquent, l'étape de compression doit permettre d'obtenir une quantité d'au moins 400 μL de gaz rares pour cette application.

Par ailleurs, on peut définir un facteur de surconcentration équivalent au taux d'augmentation de pression produit par un système de pompe seringue. Selon l'exemple, ce facteur est contraint par un volume final du gaz surconcentré d'au moins 500 μL. En partant d'une dimension de getter initiale correspondant à un volume de gaz libre $V_f^{GR}$, le taux de concentration peut être d'au maximum : $\dfrac{V_f^{GR}}{500}$ μL soit par exemple pour $V_f^{GR} = 1000$ μL, le facteur de surconcentration n'excède pas 2. Par conséquent, pour cette application, l'étape de compression permet de comprimer les gaz rares, de sorte à obtenir un volume d'au moins 500 μL avec un facteur de compression de 2.

3. Analyse des gaz rares

Afin de caractériser le fluide gazeux, on étudie les gaz rares compris dans ce fluide. Pour cette étude, on peut mettre en oeuvre une analyse des gaz rares extraits et comprimés. Selon un mode de réalisation de l'invention, cette analyse peut être mise en oeuvre par une chromatographie en phase gazeuse couplée à une spectrométrie de masse (GC-MS) et/ou en spectrométrie de masse statique (quadripolaire ou magnétique par exemple) et/ou une chromatographie en phase gazeuse couplée à une spectrométrie de masse à rapports isotopiques (GC-IRMS).

Le procédé selon l'invention trouve une application dans le domaine de l'exploitation des réservoirs géologiques comprenant des gaz naturels ($CO_2$, $N_2$, hydrocarbures, hélium) ou stockés industriellement ($CH_4$, $CO_2$, $H_2$), en effet, en fonction des résultats d'une analyse d'un échantillon de gaz prélevé dans un réservoir géologique, on peut caractériser les gaz présents dans ce réservoir, et à partir de cette caractérisation on peut définir une exploitation possible du réservoir.

L'invention concerne également un système d'analyse d'un fluide gazeux comprenant au moins un gaz rare, le système d'analyse comprenant :

- des moyens d'extraction dudit gaz rare comprenant une chambre intégrant un substrat de getterisation, ledit substrat de getterisation étant apte à piéger tous les composants dudit fluide gazeux à l'exception dudit gaz rare,
- des moyens de compression dudit gaz rare extrait, aptes à comprimer et à injecter ledit gaz rare pour ladite analyse, et
- des moyens d'analyse dudit gaz rare comprimé.

Tel que défini précédemment, le substrat de getterisation est du type getter non-évaporable pour la purification de gaz sous pression, qui par la réactivité d'un alliage métallique permet de piéger d'une manière irréversible et à volume constant les molécules gazeuses réactives présentes dans un flux de gaz inerte sous pression. Avantageusement, le substrat de getterisation est un alliage métallique sous forme de mousse, de poudre, de dépôts, de tiges, de rubans. Cette liste n'est pas exhaustive. De préférence, le substrat de getterisation est une mousse ou une poudre à base de titane, de zirconium, d'aluminium ou à base d'un alliage de ces différents métaux. En effet, ce type de substrat possède une capacité de piégeage élevée.

Le gaz extrait du fluide gazeux est ensuite comprimé par les moyens de compression, de sorte que la pression et le volume de gaz rares soient adaptés aux moyens d'analyse, c'est-à-dire aux appareils de mesure, qui sont mis en oeuvre pour déterminer, quantifier et caractériser les gaz rares présents dans le fluide gazeux. Les moyens de compression peuvent comprendre une pompe seringue de surconcentration comportant un piston, dont le déplacement peut être ajusté et commandé pour adapter le volume et la pression du gaz rare pour l'analyse. En effet, la position du piston de la pompe seringue de surconcentration permet de contrôler le volume et la pression des gaz rares.

Les moyens d'analyse peuvent être un chromatographe en phase aqueuse couplée à un spectromètre de masse (GC-MS) et/ou un spectromètre de masse statique (MS statique (quadripolaire par exemple)) et/ou un chromatographe en phase gazeuse couplée à un spectromètre de masse de rapports isotopiques (GC-IRMS).

Selon un mode de réalisation de l'invention, le fluide gazeux est un échantillon de gaz. Un tel échantillon peut être un récipient de forme sensiblement cylindrique, dont les extrémités sont équipées d'au moins une vanne pour libérer et/ou enfermer un gaz dans le récipient.

En outre, le système d'analyse selon l'invention peut comprendre une pompe seringue de transfert apte à capter le fluide gazeux et à le transférer aux moyens d'extraction dudit gaz rare, la pompe seringue de transfert comprenant un piston dont le déplacement peut être commandé pour adapter le débit d'injection dudit fluide dans lesdits moyens d'extraction dudit gaz rare.

Le système d'analyse selon l'invention peut également comprendre un restricteur de débit entre l'arrivée du fluide gazeux et les moyens d'extraction des gaz rares. Le restricteur de débit permet d'assurer un différentiel de pression entre l'arrivée du fluide gazeux et le getter. Le dimensionnement du restricteur de débit permet d'engendrer un débit vers le getter d'un ordre de grandeur similaire au taux de piégeage du getter afin d'optimiser la préconcentration et la vitesse de piégeage.

De plus, le système selon l'invention peut comporter des moyens de chauffage, par exemple un four, permettant de chauffer les moyens d'extraction des gaz rares. En effet, la température du getter joue un rôle important sur les vitesses de diffusion des particules piégées dans l'alliage du getter. On peut donc optimiser le choix de la forme du substrat de getterisation (l'alliage) ainsi que sa température de fonctionnement afin d'optimiser l'extraction des gaz rares selon un trio de critères de performance : 1) taux d'enrichissement, 2) temps de mise en oeuvre, 3) encombrement, tout en respectant le cahier des charges quantitatif (quantité d'échantillon initial disponible, quantité d'échantillon nécessaire pour l'analyse, non-contamination, etc...).

Par ailleurs, le système d'analyse selon l'invention peut comprendre des moyens de mise sous vide de la chambre apte à faire le vide dans ladite chambre intégrant ledit substrat de getterisation. Ces moyens de mise sous vide comprennent avantageusement une pompe. De plus, les moyens de mise sous vide peuvent comprendre outre un deuxième substrat de getterisation apte à piéger les molécules gazeuses réactives présentes dans une chambre sous vide poussé. Ce deuxième substrat de getterisation est disposé dans la chambre. Le vide permet d'optimiser l'efficacité du getter.

La figure 1 représente un mode de réalisation du système d'analyse selon l'invention. Selon ce mode de réalisation, le système d'analyse comporte :

- un échantillon de gaz (1) (en tant que fluide gazeux),
- une pompe seringue de transfert (2),
- un restricteur de débit (3),
- une vanne de commutation (4),
- une chambre intégrant un substrat de commutation (5),
- un four (6) en tant que moyens de chauffage,
- une pompe turbo (7) pour réaliser le vide dans la chambre (5),
- une pompe seringue de surconcentration (8),
- des moyens d'analyse (9) tels qu'un chromatographe couplé à un spectromètre de masse,
- une pompe primaire (10).

Sur cette figure, le signe P désigne un manomètre et les cercles comprenant des croix représentent des vannes.

Pour le mode de réalisation de la figure 1, on peut réaliser l'analyse de la façon suivante :

Une fois l'échantillon (1) connecté, le vide est fait dans la première partie du circuit (2, 3) via la pompe primaire (10) jusqu'à un vide de l'ordre de $10^{-2}$ mbar. Pendant cette étape, la vanne de commutation (4) met en contact le restricteur de débit (3) avec la pompe primaire (10) et le getter (5) avec le système de surconcentration (8). Le getter (5) est quant à lui pompé par la pompe turbo (7), et chauffé à 800°C au moyen du four (6), jusqu'à atteindre un vide secondaire de l'ordre de $10^{-7}$ mbar.

Ces conditions de préparation du système réunies, on isole la première partie du circuit (2, 3) de la pompe primaire (10). On ouvre la vanne du tube échantillon (1) sur cette première partie du circuit (2, 3), et on note la pression de gaz sur le manomètre. On ajuste ensuite le volume de la pompe seringue (2) en fonction de la quantité d'échantillon nécessaire, à pression atmosphérique.

Ensuite, la vanne d'isolation du getter (5) vis-à-vis du pompage (7) est fermée. La vanne de commutation (4) est basculée, ce qui permet au gaz échantillon contenu dans la première partie du circuit (2, 3) de circuler à débit contrôlé vers le getter (5). Le gaz échantillon est ainsi lentement purifié et accumulé dans le getter (5). Le gaz échantillon va se préconcentrer dans le getter (5) jusqu'à l'introduction d'une quantité jugée nécessaire de gaz dans le getter (5) (que l'on peut ajuster en fonction de la connaissance de la composition du gaz brut), mesurée par le déplacement du piston de la pompe seringue de transfert (2). Une fois la préconcentration effectuée, le chauffage du getter est éteint, et ce dernier refroidi à température ambiante.

Une fois à température ambiante, la vanne de commutation (4) est basculée, ce qui met en communication le getter (5) avec le système de surconcentration (8), préalablement mis sous vide. Le gaz est transféré vers la pompe seringue de surconcentration (8) grâce à l'action du piston. Une fois transféré, la deuxième partie du circuit (8, 9) est isolée, et le gaz présent dans la pompe seringue est comprimé par l'action du piston, jusqu'à l'obtention du volume nécessaire à la série d'analyse.

Enfin, le gaz surconcentré dans la pompe seringue (8) est injecté vers l'instrument d'analyse (9), et maintenu à pression constante durant les différentes injections grâce à l'action du piston de la pompe seringue (8).

## Revendications

1. Procédé d'analyse d'un fluide gazeux comprenant au moins un gaz rare, **caractérisé en ce qu'**on réalise les étapes suivantes :

    a) on crée le vide dans une chambre d'accumulation comprenant un substrat de getterisation, ledit substrat de getterisation étant apte à piéger les composants dudit fluide gazeux à l'exception dudit gaz rare ;
    b) on contrôle le débit dudit fluide entrant dans ladite chambre d'accumulation au moyen d'un restricteur de débit ;
    c) on extrait et on accumule ledit gaz rare dudit fluide gazeux par passage dudit fluide gazeux dans ledit substrat de getterisation de ladite chambre d'accumulation;
    d) on comprime ledit gaz rare extrait de sorte que la pression et le volume dudit gaz rare comprimé soient adaptés à la pression et au volume de ladite analyse ; et
    e) on analyse ledit gaz rare comprimé.

2. Procédé selon la revendication 1, dans lequel on analyse ledit gaz rare par chromatographie en phase gazeuse couplée à une spectrométrie de masse.

3. Procédé selon l'une des revendications précédentes, dans lequel ledit substrat de getterisation est un alliage métallique sous forme de mousse, de poudre, de dépôt, de tiges, de rubans.

4. Procédé selon la revendication 3, dans lequel ledit substrat de getterisation est une mousse ou une poudre de titane, de zirconium, d'aluminium ou d'alliage de l'un de ces métaux.

5. Procédé selon l'une des revendications précédentes, dans lequel on chauffe ledit substrat de getterisation pour l'étape d'extraction dudit gaz rare.

6. Procédé selon l'une des revendications précédentes, dans lequel préalablement à ladite étape d'extraction dudit gaz rare, on réalise le vide dans une chambre intégrant ledit substrat de getterisation.

7. Procédé selon l'une des revendications précédentes, dans lequel on contrôle le débit du passage dudit fluide gazeux dans ledit substrat de getterisation.

8. Système d'analyse d'au moins fluide gazeux comprenant au moins un gaz rare, **caractérisé en ce qu'**il comprend :

   - un restricteur de débit pour contrôler le débit dudit fluide gazeux,
   - des moyens d'extraction et d'accumulation (5) dudit gaz rare comprenant une chambre d'accumulation intégrant un substrat de getterisation, ledit substrat de getterisation étant apte à piéger tous les composants dudit fluide gazeux à l'exception dudit gaz rare,
   - des moyens de mise sous vide de ladite chambre d'accumulation,
   - des moyens de compression (8) dudit gaz rare extrait, les moyens de compression (8) étant aptes à comprimer et à injecter ledit gaz rare pour ladite analyse, et
   - des moyens d'analyse (9) dudit gaz rare comprimé.

9. Système selon la revendication 8, dans lequel ledit fluide gazeux est un échantillon (1) de gaz prélevé dans un réservoir géologique.

10. Système selon l'une des revendications 8 ou 9, dans lequel lesdits moyens d'analyse (9) dudit gaz rare comprennent un chromatographe couplé à un spectromètre de masse.

11. Système selon l'une des revendications 8 à 10, dans lequel ledit substrat de getterisation est un alliage métallique sous forme de mousse, de poudre, de dépôt, de tiges ou de rubans.

12. Système selon la revendication 11, dans lequel ledit substrat de getterisation est une mousse ou une poudre de titane, de zirconium, d'aluminium ou d'alliage de l'un de ces métaux.

13. Système selon l'une des revendications 8 à 12, dans lequel ledit système d'analyse comprend en outre des moyens de chauffage (6) de ladite chambre (5) intégrant ledit substrat de getterisation.

14. Système selon l'une des revendications 8 à 13, dans lequel ledit système d'analyse comprend en outre des moyens de mise sous vide de ladite chambre (5) apte à faire le vide dans ladite chambre (5) intégrant ledit substrat de getterisation, lesdits moyens de mise sous vide comprenant au moins une pompe (7).

15. Système selon la revendication 14, dans lequel lesdits moyens de mise sous vide comprennent en outre un deuxième substrat de getterisation apte à piéger les molécules gazeuses réactives présentes dans ladite chambre sous vide poussé.

16. Système selon l'une des revendications 8 à 15, dans lequel lesdits moyens de compression dudit gaz rare comprennent une pompe seringue de surconcentration (8) comportant un piston dont le déplacement peut être commandé pour adapter le volume et la pression dudit gaz rare pour ladite analyse.

17. Système selon l'une des revendications 8 à 16, dans lequel ledit système d'analyse comprend une pompe seringue de transfert (2) apte à capter ledit fluide gazeux et à le transférer auxdits moyens d'extraction dudit gaz rare, ladite pompe seringue de transfert (2) comprenant un piston dont le déplacement peut être commandé pour adapter le débit d'injection dudit fluide dans lesdits moyens d'extraction (5) dudit gaz rare.

18. Système selon l'une des revendications 8 à 17, dans lequel ledit système d'analyse comporte un restricteur de débit (3) en amont des moyens d'extraction (5) dudit gaz rare.

**Figure 1**

Europäisches
Patentamt

European
Patent Office

Office européen
des brevets

**RAPPORT DE RECHERCHE EUROPEENNE**

Numéro de la demande

EP 14 30 5326

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | US 4 833 915 A (RADD FREDERICK J [US] ET AL) 30 mai 1989 (1989-05-30) | 1-3, 6-11, 14-18 | INV. G01N33/00 |
| Y | * le document en entier * | 4,5,12, 13 | |
| Y | EP 0 475 312 A2 (JAPAN PIONICS [JP]) 18 mars 1992 (1992-03-18) | 4,5,12, 13 | |
| A | * page 3, ligne 28 - page 4, ligne 43 * | 1-3, 6-11, 14-18 | |
| A | GB 961 925 A (WESTERN DETAIL MANUFACTURERS L) 24 juin 1964 (1964-06-24) * le document en entier * | 1-18 | |
| A | US 3 447 360 A (LASETER JOHN L) 3 juin 1969 (1969-06-03) * colonne 4, ligne 48 - colonne 6, ligne 73; figure 1 * | 1-18 | |
| A | US 5 545 894 A (FUNSTEN HERBERT O [US] ET AL) 13 août 1996 (1996-08-13) * abrégé; figure 1 * | 1-18 | DOMAINES TECHNIQUES RECHERCHES (IPC) G01N C01B E21B H01J |

Le présent rapport a été établi pour toutes les revendications

| Lieu de la recherche | Date d'achèvement de la recherche | Examinateur |
|---|---|---|
| La Haye | 22 avril 2014 | Gilow, Christoph |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 14 30 5326

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-04-2014

| Document brevet cité au rapport de recherche | | Date de publication | Membre(s) de la famille de brevet(s) | | Date de publication |
|---|---|---|---|---|---|
| US 4833915 | A | 30-05-1989 | GB<br>NO<br>US | 2213174 A<br>885388 A<br>4833915 A | 09-08-1989<br>05-06-1989<br>30-05-1989 |
| EP 0475312 | A2 | 18-03-1992 | DE<br>DE<br>EP<br>JP<br>JP<br>US<br>US | 69124172 D1<br>69124172 T2<br>0475312 A2<br>H054809 A<br>2980425 B2<br>5194233 A<br>5294422 A | 27-02-1997<br>30-04-1997<br>18-03-1992<br>14-01-1993<br>22-11-1999<br>16-03-1993<br>15-03-1994 |
| GB 961925 | A | 24-06-1964 | AUCUN | | |
| US 3447360 | A | 03-06-1969 | AUCUN | | |
| US 5545894 | A | 13-08-1996 | AUCUN | | |

EPO FORM P0460

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4833915 A
- WO 2010049608 A
- FR 2938068